# EUROPÄISCHE PATENTSCHRIFT

(11) **EP 0 986 963 B1**
(45) Veröffentlichungstag und Bekanntmachung des Hinweises auf die Patenterteilung: **07.12.2016**
(21) Anmeldenummer: 99117236.2
(22) Anmeldetag: 02.09.1999
(51) Int. Cl.: C09B 61/00, A61K 31/01, C07C 11/28, A61K 8/31, A61Q 1/12, A61K 8/67

(54) **Stabile, pulverförmige Lycopin-Formulierungen, enthaltend Lycopin mit einem Kristallinitätsgrad von grösser 20%**
Stable, powdery lycopene formulations containing lycopene with a degree of crystallisation of more than 20%
Formulations de lycopène stable et pulvérulente contenant de lycopène avec un taux de crystallisation de plus de 20%

(30) Priorität: 14.09.1998 DE 19841930
(43) Veröffentlichungstag der Anmeldung: 22.03.2000
(73) Patentinhaber: BASF SE, 67056 Ludwigshafen am Rhein (DE)
(72) Erfinder: Runge, Frank, Dr., 67133 Maxdorf (DE); Auweter, Helmut, Dr., 67117 Limburgerhof (DE); Musaeus-Jensen, Nina, 2900 Hellerup (DK); Haberkorn, Herbert, Dr., 67269 Grünstadt (DE); Rieger, Jens, Dr., 67069 Ludwigshafen (DE)
(74) Vertreter: BASF IP Association

(56) Entgegenhaltungen:
- EP-A- 0 065 193
- EP-A- 0 410 236
- EP-A- 0 832 569
- WO-A-96/13178

## Beschreibung

Die Erfindung betrifft stabile, pulverförmige Lycopin-Formulierungen, enthaltend Lycopin mit einem Kristallinitätsgrad von größer 20%, Verfahren zu deren Herstellung sowie ihre Verwendung als Zusatz zu Lebensmitteln, Kosmetika, Pharmazeutika und Tierfuttermitteln.

Lycopin, das zur Stoffklasse der Carotinoide gehört, kommt in der Natur weitverbreitet vor. So bilden Tomaten mit einem Lycopingehalt von ca. 20 mg/kg Tomate die wichtigste natürliche Quelle dieses Rotpigments.

Epidemiologische Studien haben gezeigt, daß ein häufiger und regelmäßiger Verzehr von Tomaten oder Tomatenprodukten das Risiko chronischer Erkrankungen, u.a. Herz- und Kreislauferkrankungen, mindert sowie einen positiven Einfluß auf die Krebsprävention ausübt. Diese Schutzfunktion des Lycopins wird in seiner Wirkung als sehr effektives Antioxidants gesehen.

Sowohl für die Lebensmittel- und Futtermittelindustrie als auch für die pharmazeutische Technologie stellt Lycopin z.B. als Ersatz für künstliche Farbstoffe einen wichtigen Farbkörper dar und ist darüber hinaus aus den eingangs genannten Gründen als Lebensmittelzusatzstoff für die Gesundheitsvorsorge von Interesse.

Die Synthese von Lycopin ist u.a. beschrieben in EP-A-382067 und EP-A-000140. WO 97/48287 beschreibt die Extraktion von Lycopin als natürliches Carotinoid aus Tomaten.

Wie alle Carotinoide ist auch Lycopin in Wasser unlöslich, während in Fetten und Ölen eine jedoch nur geringe Löslichkeit gefunden wird. Diese begrenzte Löslichkeit sowie die hohe Oxidationsempfindlichkeit stehen einer direkten Anwendung des relativ grobkörnigen kristallinen Lycopins in der Einfärbung von Lebens- und Futtermitteln entgegen, da die Reinsubstanz in grobkristalliner Form zu instabil ist, nur unzureichend resorbiert wird und somit nur schlechte Färbungsergebnisse liefert.

Nur durch gezielt hergestellte Formulierungen, in denen die Wirkstoffe in fein verteilter Form und gegebenenfalls durch Schutzkolloide oxidationsgeschützt vorliegen, lassen sich bei der direkten Einfärbung von Lebensmitteln verbesserte Farbausbeuten erzielen.

Im Hinblick auf die, im Vergleich zu anderen Carotinoiden, besonders geringe Oxidationsstabilität und die damit auch verbundene geringe Farbstabilität bzw. Lagerstabilität von Lycopin, werden an diese Formulierungen besonders hohe Ansprüche gestellt.

Für Carotinoide allgemein sind eine Vielzahl von Formulierungen sowie Verfahren zu ihrer Herstellung beschrieben.

So ist Lycopin beispielsweise unter dem Namen Lyc-O-Mato^{®} (Fa. LycoRed, Israel) als 6 %ige ölige Dispersion erhältlich. Es wird gemäß WO 97/48287 als natürliches Carotinoid aus Tomaten extrahiert. Aufgrund des hohen Phospholipidanteils im Lyc-O-Mato^{®}, verbunden mit einer hohen Viskosität der öligen Dispersion, sind die anwendungstechnischen Eigenschaften dieser Formulierung, u.a. die Wasserdispergierbarkeit, nicht zufriedenstellend.

EP-A-0 410 236 beschreibt ein Verfahren zur Herstellung von Carotinoid Trockenpulvern, in dem man eine Suspension eines Carotinoids in einem hochsiedenden Öl kurzzeitig erhitzt, diese Mischung aus geschmolzenem Carotinoid und Öl in eine wäßrige Lösung eines Kolloids emulgiert und diese Emulsion anschließend sprühtrocknet.

In DE-A-1 211 911 werden Carotinoidpräparate beschrieben, für deren Herstellung zunächst die Carotinoide in einem in Wasser unlöslichen Lösungsmittel gelöst werden, diese Lösung anschließend in eine wäßrige Schutzkolloidlösung einemulgiert und sprühgetrocknet wird.

EP-A-0 065 193 beschreibt ein Verfahren zur Herstellung von pulverförmigen Carotinoidpräparaten, das dadurch gekennzeichnet ist, daß man ein Carotinoid in einem mit Wasser mischbaren, organischen Lösungsmittel bei erhöhten Temperaturen kurzzeitig löst, aus der erhaltenen Lösung sofort durch schnelles Mischen mit einer wäßrigen Lösung eines Schutzkolloids das Carotinoid in kolloid-disperser Form ausfällt und die erhaltene Dispersion in ein Trockenpulver überführt.

Gemäß EP-A-0 832 569 erhält man durch Tempern der nach EP-A-0 065 193 hergestellten Dispersion bei einer Temperatur zwischen 40°C und 90°C und anschließende Sprühtrocknung ein Carotinoid Trockenpulver, in dem die Wirkstoffpartikel weitgehend röntgenamorph vorliegen.

WO 98/16204 beschreibt ein Verfahren zur Herstellung von pulverförmigen Carotinoidpräparaten, das dadurch gekennzeichnet ist, daß man ein Carotinoid im Autoklav unter erhöhtem Druck und Temperatur in Dimethylether löst und durch sehr schnelles Entspannen das Lösungsmittel wieder abtrennt.

Zahlreiche Methoden, u.a. beschrieben in Chimia 21, 329 (1967), WO 91/06292 sowie in WO 94/19411, bedienen sich dabei der Vermahlung von Carotinoiden, z.B. ß-Carotin mittels einer Kolloidmühle und erzielen damit Partikelgrößen von 2 bis 10 µm.

Trotz der Vielzahl an Veröffentlichungen auf diesem Gebiet, finden sich in keiner der im Stand der Technik genannten Schriften Hinweise zur Lösung des Stabilitätsproblems von Lycopin, die über allgemeine Aussagen zu Carotinoid-Formulierungen hinausgehen, mit einer Ausnahme.

Nach der Lehre der WO 96/13178 A1 wird aus Tomaten Lycopin isoliert. Dabei entsteht ein gefriergetrocknetes Lycopin in kristalliner Form. Dieses ist nicht stabil und wird daher in einem Nicht-Lösemittel dispergiert, wodurch man ein flüssiges, stabiles Lycopin-Konzentrat erhält. Die '178 Schrift lässt sich jedoch nicht darüber aus, wie der Fachmann zu einer pulverförmigen, stabilen Formulierung enthaltend Lycopin gelangen kann.

Es war daher die Aufgabe, pulverförmige Lycopin-Formulierungen bereitzustellen, in denen das Lycopin besonders oxidations- und lichtstabil ist und die den oben genannten Nachteil des Standes der Technik nicht aufweisen.

Diese Aufgabe wurde erfindungsgemäß gelöst durch stabile, pulverförmige Lycopin-Formulierungen, enthaltend Lycopin mit einem Kristallinitätsgrad von größer 20% und mindestens ein Schutzkolloid.

Dieses mindestens eine Schutzkolloid ist ausgewählt aus der Gruppe Gelatine, Fischgelatine, Stärke, Dextrin, Pflanzenproteine, Pektin, Gummi-Arabicum, Kasein und/oder Kaseinat.

In einer fortgeführten Ausführungsform der Erfindung enthält die Lycopinformulierung mindestens Fischgelatine als Schutzkolloid.

Der Kristallinitätsgrad von Lycopin in den erfindungsgemäßen Formulierungen läßt sich beispielsweise durch Röntgenbeugungsmessungen bestimmen und liegt im allgemeinen im Bereich größer 20%, bevorzugt im Bereich von 25 bis 100%, besonders bevorzugt im Bereich von 30 bis 95%, ganz besonders bevorzugt im Bereich von 40 bis 80%.

Das in den pulverförmigen Formulierungen enthaltene Lycopin kann bezüglich der Doppelbindungsisomerie ohne Einschränkung in allen isomeren Formen vorliegen, beispielsweise in der all-trans-, 5-cis- oder 9,13-di-cis-Form. Bevorzugt sind solche pulverförmigen Formulierungen, enthaltend Lycopin mit einem all-trans Gehalt von mindestens 50%, besonders bevorzugt Lycopin mit einem all-trans Gehalt von 52 bis 100%, ganz besonders bevorzugt Lycopin mit einem all-trans Gehalt von 55 bis 90%.

Der Gehalt an Lycopin in den erfindungsgemäßen Formulierungen liegt im Bereich von 0,5 bis 25 Gew.-%, bevorzugt im Bereich von 2 bis 21 Gew.-%, besonders bevorzugt im Bereich von 5 bis 16 Gew.-%, ganz besonders bevorzugt im Bereich von 8 bis 12 Gew.-%, bezogen auf die Trockenmasse der Formulierungen.

Die mittlere Teilchengröße von Lycopin in den Trockenpulvern liegt im Bereich kleiner 10 µm, bevorzugt im Bereich kleiner 5,0 µm, besonders bevorzugt im Bereich von 0,05 bis 1 µm.

Zur Erhöhung der Stabilität der pulverförmigen Lycopin-Formulierungen ist es vorteilhaft, zusätzlich zum Wirkstoff Schutzkolloide, Weichmacher und/oder Stabilisatoren in die Formulierung einzuarbeiten.

Als Schutzkolloide werden beispielsweise Gelatine, Fischgelatine, Stärke, Dextrin, Pflanzenproteine, Pektin, Gummi-Arabikum, Kasein, Kaseinat oder Mischungen davon verwendet. Es können aber auch Polyvinylalkohol, Polyvinylpyrrolidon, Methylcellulose, Carboxymethylcellulose, Hydroxypropylcellulose und Alginate eingesetzt werden. Bezüglich näherer Einzelheiten wird auf R.A. Morton, Fat Soluble Vitamins, Intern. Encyclopedia of Food and Nutrition, Bd.9, Pergamon Press 1970, S. 128-131, verwiesen. Zur Erhöhung der mechanischen Stabilität des Endproduktes ist es zweckmäßig, dem Kolloid einen Weichmacher zuzusetzen, wie Zucker oder Zuckeralkohole, z.B. Saccharose, Glucose, Lactose, Invertzucker, Sorbit, Mannit oder Glycerin.

Das Verhältnis Schutzkolloid und Weichmacher zu Carotinoidlösung wird im allgemeinen so gewählt, daß ein Endprodukt erhalten wird, das zwischen 0,5 und 25 Gew.-% Lycopin, 10 bis 50 Gew.-%, bevorzugt 15 bis 35 Gew.-% eines Schutzkolloids, 20 bis 70 Gew.-%, bevorzugt 30 bis 60 Gew.-% eines Weichmachers, alle Prozentangaben bezogen auf die Trockenmasse des Pulvers, sowie gegebenenfalls untergeordnete Mengen eines Stabilisators enthält.

Zur Erhöhung der Stabilität des Wirkstoffes gegen oxidativen Abbau kann es vorteilhaft sein, 0 bis 10 Gew.-%, bevorzugt 0,5 bis 7,5 Gew.-%, bezogen auf die Trockenmasse der Formulierung, eines oder mehrerer Stabilisatoren wie α-Tocopherol, t-Butyl-hydroxytoluol, t-Butylhydroxyanisol, Ascorbinsäure oder Ethoxyquine zuzusetzen.

Weiterhin können Emulgatoren beispielsweise Ascorbylpalmitat, Polyglycerin-Fettsäureester, Sorbitan-Fettsäureester, Propylenglycol-Fettsäureester oder Lecithin in einer Konzentration von 0 bis 200 Gew.%, vorzugsweise 5 bis 150 Gew.%, besonders bevorzugt 10 bis 80 Gew.%, bezogen auf Lycopin verwendet werden.

Unter Umständen kann es auch vorteilhaft sein, zusätzlich ein physiologisch zugelassenes Öl wie beispielsweise Sesamöl, Maiskeimöl, Baumwollsaatöl, Sojabohnenöl oder Erdnußöl sowie Ester mittelkettiger pflanzlicher Fettsäuren in einer Konzentration von 0 bis 500 Gew.%, vorzugsweise 10 bis 300 Gew.%, besonders bevorzugt 20 bis 100 Gew.%, bezogen auf Lycopin zu verwenden.

Neben Lycopin können die Formulierungen noch weitere Carotinoide enthalten, wie z.B. ß-Carotin, Bixin, Zeaxanthin, Cryptoxanthin, Citranaxanthin, Canthaxanthin, ß-Apo-4-carotinal, ß-Apo-8-carotinal, ß-Apo-8-carotinsäureester, Astaxanthin oder Lutein, einzeln oder als Mischung.

Gegenstand der Erfindung ist auch ein Verfahren zur Herstellung der eingangs beschriebenen, stabilen, pulverförmigen Lycopin-Formulierungen, enthaltend Lycopin mit einem Kristallinitätsgrad von größer 20%, dadurch gekennzeichnet, daß man
a) eine molekulardisperse Lösung von Lycopin gegebenenfalls zusammen mit einem Emulgator und/oder einem eßbaren Öl in einem mit Wasser mischbaren, organischen Lösungsmittel oder einer Mischung aus Wasser und einem mit Wasser mischbaren, organischen Lösungsmittel bei Temperaturen größer 30°C herstellt,
b) diese Lösung mit einer wäßrigen Lösung eines Schutzkolloids versetzt, wobei die hydrophile Lösungsmittelkomponente in die wäßrige Phase überführt wird und die hydrophobe Phase des Carotinoids als nanodisperse Phase entsteht,
c) und die gebildete Dispersion für die Herstellung eines wasserdispergierbaren Trockenpulvers von dem Lösungsmittel und dem Wasser befreit und, gegebenenfalls in Gegenwart eines Überzugsmaterials, trocknet.

Zur Durchführung des erfindungsgemäßen Verfahrens sind vor allem wassermischbare, thermisch stabile, flüchtige, nur Kohlenstoff, Wasserstoff und Sauerstoff enthaltene Lösungsmittel wie Alkohole, Ether, Ester, Ketone und Acetale geeignet. Vorzugsweise werden Ethanol, n-Propanol, Isopropanol, 1,2-Butandiol-1-methylether, 1,2-Propandiol-1-n-propylether oder Aceton verwendet. Allgemein verwendet man zweckmäßig solche Lösungsmittel, die mindestens zu 10% wassermischbar sind, einen Siedepunkt unter 200°C aufweisen und/oder weniger als 10 Kohlenstoffe haben.

In einer bevorzugten Ausführungsform des Verfahren wird im Schritt a) die molekulardisperse Lycopinlösung bei Temperaturen von 50°C bis 240°C, besonders bevorzugt bei Temperaturen von 140°C bis 180°C hergestellt und unmittelbar anschließend im Schritt b) mit der wäßrigen Lösung des Schutzkolloids versetzt, wobei sich eine Mischungstemperatur von etwa 35°C bis 80°C, besonders bevorzugt von 55°C bis 70°C einstellt.

Da die Einwirkung hoher Temperaturen den gewünschten hohen all-trans Isomerenanteil herabsetzen kann, löst man das Lycopin möglichst rasch, beispielsweise im Sekundenbereich, z.B. in 0,1 bis 10 Sekunden, besonders bevorzugt in weniger als 1 Sekunde. Zur raschen Herstellung der molekulardispersen Lösung kann die Anwendung von erhöhtem Druck, z.B. im Bereich von 20 bar bis 100 bar, vorzugsweise 30 bis 80 bar, vorteilhaft sein.

Je nach Art und Menge des verwendeten Schutzkolloids erhält man nach dem Verfahrensschritt b) eine tiefgefärbte viskose Flüssig-keit. Die Entfernung des Lösungsmittels kann beispielsweise durch Extraktion mit einem mit Wasser nicht mischbaren Lösungsmittel oder, je nach Siedepunkt in an sich bekannter Weise, z.B. durch Destillation, gegebenenfalls unter vermindertem Druck, erfolgen. In diesem Fall hat es sich als zweckmäßig und möglich erwiesen, das bei Verwendung von Isopropanol erhaltene Azeotrop ohne Wasserentfernung unmittelbar als Lösungsmittel einzusetzen. Vorzugsweise erfolgt die Lösungsmittelabtrennung jedoch gemeinsam mit der Entfernung des Wassers durch Sprühtrocknung oder Sprühgranulation.

Hinsichtlich einer näheren Verfahrens- und Apparatebeschreibung wird an dieser Stelle ausdrücklich auf EP-A-0 065 193 Bezug genommen.

Die nach dem obigen Verfahren hergestellten Lycopin-Trockenpulver zeichnen sich durch eine sehr gute Redispergierbarkeit, sowohl im warmen als auch im kalten Wasser aus.

Es wurde nun gefunden, daß eine nach dem oben genannten Verfahren hergestellte pulverförmige Lycopin-Formulierung, im Vergleich zu entsprechenden ß-Carotin- oder Canthaxanthin-Formulierungen überraschende Eigenschaften besitzt. Während ß-Carotin und Canthaxanthin in einem gemäß EP-A-0 065 193 hergestellten Trockenpulver mit einem Kristallinitätsgrad von nur 10% überwiegend amorph vorliegen, zeigt der anhand von Röntgenbeugungsdiagrammen ermittelte Kristallinitätsgrad von Lycopin in der Zubereitung Werte im Bereich größer 20%, bevorzugt im Bereich von 25 bis 100%, besonders bevorzugt im Bereich von 30 bis 95%, ganz besonders bevorzugt im Bereich von 40 bis 80%.

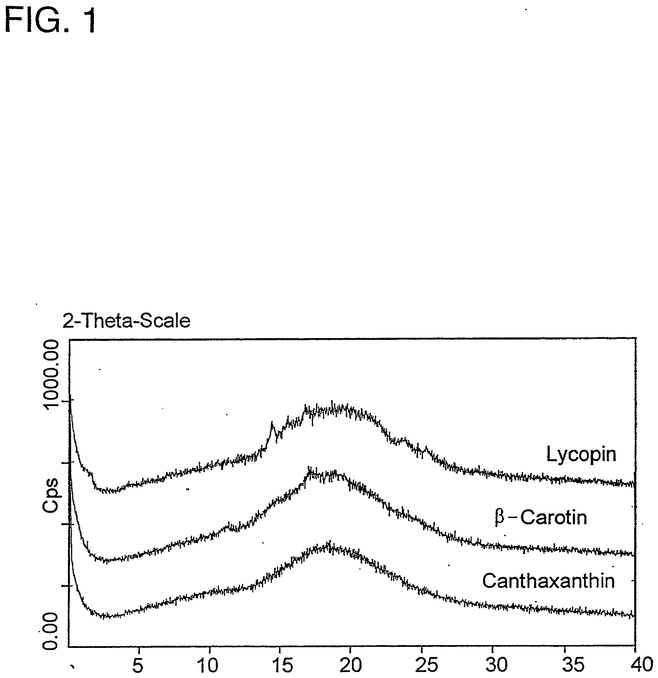

In FIG. 1 sind die jeweiligen Röntgenbeugungsdiagramme der in Tabelle 1 beschriebenen Trockenpulver von Lycopin, ß-Carotin und Canthaxanthin abgebildet. Die Röntgenweitwinkeldiagramme wurden im Winkelbereich 1° ≤ 20 ≤ 40° registriert. Die Bestimmung der Wirkstoffkristallinität W_{cw} erfolgte in dem Diagrammausschnitt 11° ≤ 2Θ ≤ 31°, in dem der Großteil der Wirkstoffinterferenz auftritt, nach der Formel W_{cw} = F_{cw}/F₁₀₀·C_{w}. F₁₀₀ ist dabei die unter vergleichbaren Bedingungen gemessene und im gleichen Winkelbereich planimetrierte kristalline Streuung des reinen Wirkstoffs und c_{w} die Konzentration des Wirkstoffs in der Formulierung.

Überraschend war auch, daß sich die so hergestellten erfindungsgemäßen Lycopin-Formulierungen im photochemischen Stabilitätstes im Vergleich zu entsprechenden ß-Carotin- und Canthaxanthin-Formulierungen durch eine besondere Lichtstabilität auszeichnen (siehe Tabelle 1).

**Tabelle 1:**

| Trockenpulver von:¹⁾ | Gehalt [Gew.-%] | all-trans Gehalt [%] | Kristallinitätsgrad [%] | Halbwertszeit² [min] |
|---|---|---|---|---|
| Lycopin | 10 | 72 | 64 | 160 |
| ß-Carotin | 10 | 78 | 10 | 28 |
| Canthaxanthin | 10 | 73 | 0 | 34 |

| | | | | |
|---|---|---|---|---|
| ¹⁾ Hergestellt gemäß Beispiel 1 ²⁾ Bestrahlungszeit, nach der im Lichtstabilitätstest³⁾ die anfängliche Extinktion im Absorptionsmaximum auf die Hälfte abgenommen hat. ³⁾ Im Lichtstabilitätstest wurde das jeweils zu untersuchende Trockenpulver in VE-Wasser dispergiert, mit Zitronensäure auf pH 3 gestellt und in 50 ml Quarzampullen unter Luftzutritt im Suntest CPS Gerät (Fa. Heraeus) bestrahlt. Gemessen wurde das UV/VIS-Spektrum als Funktion der Bestrahlungszeit. | | | | |

Gegenstand der Erfindung ist darüberhinaus ein weiteres Verfahren zur Herstellung von stabilen, pulverförmigen Lycopin-Formulierungen, enthaltend Lycopin mit einem Kristallinitätsgrad von größer 20%, dadurch gekennzeichnet, daß man kristallines Lycopin mit einem all-trans Isomerengehalt von mindestens 50% in einem wäßrigen Medium in Anwesenheit eines Schutzkolloids mahlt und die so erhaltene Lycopinsuspension für die Herstellung eines Trockenpulvers trocknet. Das so erhaltene Trockenpulver läßt sich beispielsweise zu Färbezwecken wiederum gut in Wasser redispergieren.

Hinsichtlich einer näheren Verfahrens- und Apparatebeschreibung sei an dieser Stelle ausdrücklich auf EP-A-0 498 824 Bezug genommen.

Überraschenderweise konnte bei dem oben beanspruchten Mahlverfahren zur Herstellung von Lycopin-Trockenpulvern der Kristallinitätsgrad von Lycopin durch Variation der Mahldauer und/oder Mahltemperatur eingestellt werden.

Neben den bereits genannten vorteilhaften Farbstabilitäten der erfindungsgemäßen Lycopin-Trockenpulver, konnten ebenfalls sehr gute Lagerstabilitäten beobachtet werden. So zeigt beispielsweise eine nach dem Mahlverfahren hergestellte Lycopin-haltige Formulierung eine hervorragende Lagerstabilität in pharmazeutischen Präparaten, beispielsweise in Multivitamintabletten (siehe Tabelle 2).

**Tabelle 2:**

| Trockenpulver von: ¹⁾ | Gehalt [Gew.-%] | Kristallinitätsgrad [%] | Gehalt nach 3 Monaten Lagerzeit²⁾ [%] |
|---|---|---|---|
| Lycopin | 10 | 33 | 89 |
| Lycopin | 10 | 25 | 80 |
| Lycopin | 10 | 19 | 60 |

| | | | |
|---|---|---|---|
| ¹⁾ Hergestellt gemäß Beispiel 3 ²⁾ Lagerstabilität von Lycopin in Multivitaminmineraltabletten, Lagerung bei 40°C und 75% Luftfeuchtigkeit | | | |

Neben den erfindungsgemäßen Verfahren zur Herstellung der Lycopin-Formulierung sind gegebenenfalls auch andere, u.a. die als Stand der Technik eingangs zitierten Herstellverfahren denkbar, beispielsweise ein kombiniertes Emulgier- und Sprühtrocknungsverfahren.

Gegenstand der Erfindung sind auch stabile, wäßrige Lycopin-Dispersionen, enthaltend Lycopin mit einem Kristallinitätsgrad von größer 20%.

Diese Dispersionen erhält man beispielsweise dadurch, daß man bei dem Verfahren zur Herstellung der erfindungsgemäßen Lycopin-Trokkenpulver auf den Trocknungsschritt, z.B. die Sprühtrocknung verzichtet.

Ebenso wie die pulverförmigen Lycopin-Formulierungen zeigen die wäßrigen Lycopin-Dispersionen eine sehr gute Lichtstabilität.

Die erfindungsgemäßen Lycopin-Formulierungen eignen sich u.a. als Zusatzstoff für Lebensmittelzubereitungen, als Mittel für die Herstellung pharmazeutischer und kosmetischer Zubereitungen sowie für die Herstellung von Nahrungsergänzungspräparaten im Human- und Tierbereich.

Typische Einsatzgebiete im Lebensmittelbereich sind beispielsweise die Färbung von Getränken, Milchprodukten wie Joghurt, Milchmixgetränken oder Milchspeiseeis sowie von Puddingpulvern, Eiprodukten, Backmischungen und Süßwaren.

Im Futtermittelbereich können die Lycopin-Formulierungen beispielsweise zur Eidotter- und Broilerhautpigmentierung in der Geflügelzucht verwendet werden.

Im Kosmetikbereich können die Lycopin-Trockenpulver beispielsweise als Farbmittel für dekorative Körperpflegemittel verwendet werden.

Unter Nahrungsergänzungspräparate sowie pharmazeutische Zubereitungen, die die erfindungsgemäße Lycopin-Formulierung enthalten, sind u.a. Tabletten, Dragees sowie Hart- und Weichgelatinekapseln zu verstehen.

Kosmetische Zubereitungen, die die erfindungsgemäßen Lycopin-Formulierungen enthalten können, sind beispielsweise topisch anwendbare Zubereitungen, insbesondere dekorative Körperpflegemittel wie Lippenstifte, Gesichts-Make-up in Form einer Creme, einer Lotion, eines Puders oder auch als Rouge.

In den nachfolgenden Beispielen wird die Herstellung der erfindungsgemäßen Lycopin-Formulierungen näher erläutert.

### Beispiel 1

### Herstellung eines 10 %igen Lycopin-Trockenpulvers

25 g krist. Lycopin wurden in einer Lösung von 4,6 g Ascorbylpalmitat und 3,6 g α-Tocopherol in 300 g Isopropanol suspendiert. Diese Suspension wurde bei einer Förderrate von 2,0 kg/h und 50 bar Systemdruck mit 400 g Isopropanol gemischt (Förderrate 2,6 kg/h), das mittels Wärmetauscher erhitzt wurde. Die dabei bei einer Temperatur von 170°C entstehende molekulardisperse Lösung wurde in einer Mischkammer turbulent mit einer mit 30,5 kg/h geförderten Lösung von 72,4 g Fischgelatine und 108 g Glucosesirup in 4776 g Wasser vermischt, wobei eine Dispersion einer mikrodispersen Lycopinphase in einer Wasser/Isopropanol-Mischung gebildet wurde (Lycopingehalt 0,3 %). Die Dispersion wurde in einem Vakuumverdampfer aufkonzentriert (Lycopingehalt 3,0 %), wobei das Isopropanol abgetrennt wurde und anschließend mittels Sprühtrocknung in ein Trockenpulver überführt. Der photometrisch bestimmte Gehalt an Lycopin betrug 9,8 %.

Durch quasi-elastische Lichtstreuung wurde die mittlere Teilchengröße bestimmt zu 134 nm. Der mit HPLC bestimmte all-trans-Gehalt betrug 72 %; der mittels Röntgenweitwinkelstreuung bestimmte Kristallinitätsgrad des Lycopins betrug 64 %.

### Beispiel 2

### Herstellung eines 20 %igen Lycopin-Trockenpulvers

50 g krist. Lycopin wurden in einer Lösung von 4,6 g Ascorbylpalmitat und 3,6 g α-Tocopherol in 275 g Isopropanol suspendiert. Diese Suspension wurde bei einer Förderrate von 2,0 kg/h bei 50 bar Systemdruck mit 433 g Isopropanol gemischt (Förderrate 2,6 kg/h), das mittels Wärmetauscher erhitzt wurde. Die dabei bei einer Temperatur von 170°C entstandene molekulardisperse Lösung wurde in einer Mischkammer turbulent mit einer mit 30,5 kg/h geförderten, Lösung von 72,4 g Fischgelatine und 108 g Glucosesirup in 4773 g Wasser vermischt, wobei eine Dispersion einer mikrodispersen Lycopinphase in einer Wasser/Isopropanol-Mischung gebildet wurde. Die Dispersion wurde in einem Vakuumverdampfer aufkonzentriert (Lycopingehalt 4,8 %) und anschließend mittels Sprühtrocknung in ein Trockenpulver überführt. Der photometrisch bestimmte Gehalt an Lycopin betrug 20 %.

Durch quasi-elastische Lichtstreuung wurde die mittlere Teilchengröße bestimmt zu 244 nm. Der mit HPLC bestimmte all-trans-Gehalt betrug 52 %; der mittels Röntgenweitwinkelstreuung bestimmte Kristallinitätsgrad des Lycopins betrug 51 %.

### Beispiel 3

### Mahlung von kristallinem Lycopin

Unter Schutzgas wurden 15 g kristallines Lycopin zu einer auf 60°C erwärmten Lösung von 21 g 240 Bloom A Gelatine und 1,5 g Natriumascorbat in 150 ml Wasser gegeben. Die Suspension wurde mittels einer Kugelmühle (DynoMill^{®} Typ KDL) 7 Stunden gemahlen. Nach Zugabe von 20 g Gelatine, 35 g Saccharose und 1,5 g Tocopherol wurde die Suspension sprühgetrocknet. Man erhielt ein Trokkenpulver mit einem Lycopingehalt von 10 Gew.-%. Der mit HPLC bestimmte all-trans-Gehalt betrug 91 %; der mittels Röntgenweitwinkelstreuung bestimmte Kristallinitätsgrad des Lycopins betrug 33%.

## Patentansprüche

1. Stabile, pulverförmige Lycopin-Formulierungen, enthaltend Lycopin mit einem Kristallinitätsgrad von größer 20% und mindestens ein Schutzkolloid.

2. Lycopinformulierung nach Anspruch 1 enthaltend mindestens ein Schutzkolloid ausgewählt aus der Gruppe Gelatine, Fischgelatine, Stärke, Dextrin, Pflanzenproteine, Pektin, Gummi-Arabicum, Kasein und/oder Kaseinat.

3. Lycopinformulierung nach Anspruch 1 oder 2, enthaltend mindestens Fischgelatine als Schutzkolloid.

4. Lycopin-Formulierungen nach einem der Ansprüche 1 bis 3, enthaltend Lycopin mit einem all-trans Isomerengehalt von mindestens 50%.

5. Lycopin-Formulierungen nach einem der Ansprüche 1 bis 4, enthaltend 0,5 bis 25 Gew.-% Lycopin, 10 bis 50 Gew.-% eines Schutzkolloids, 20 bis 70 Gew.-% eines Weichmachers, 0 bis 10 Gew.-% eines Stabilisators, alle Prozentangaben bezogen auf die Trockenmasse des Pulvers.

6. Lycopin-Formulierungen nach einem der Ansprüche 1 bis 5, enthaltend Lycopin mit einer Teilchengröße von weniger als 10 µm.

7. Lycopin-Formulierungen nach einem der Ansprüche 1 bis 6, **dadurch gekennzeichnet, dass** sie wasserdispergierbar sind.

8. Verfahren zur Herstellung von stabilen, pulverförmigen Lycopin-Formulierungen, definiert gemäß Anspruch 1, **dadurch gekennzeichnet, dass** man
a) eine molekulardisperse Lösung von Lycopin gegebenenfalls zusammen mit einem Emulgator und/oder einem eßbaren Öl in einem mit Wasser mischbaren, organischen Lösungsmittel oder einer Mischung aus Wasser und einem mit Wasser mischbaren, organischen Lösungsmittel bei Temperaturen größer 30°C herstellt,
b) diese Lösung mit einer wäßrigen Lösung eines Schutzkolloids versetzt, wobei die hydrophile Lösungsmittelkomponente in die wäßrige Phase überführt wird und die hydrophobe Phase des Lycopins als nanodisperse Phase entsteht,
c) und die gebildete Dispersion für die Herstellung eines wasserdispergierbaren Trockenpulvers von dem Lösungsmittel und dem Wasser befreit und, gegebenenfalls in Gegenwart eines Überzugsmaterials, trocknet.

9. Verfahren nach Anspruch 8, **dadurch gekennzeichnet, dass** man im Schritt a) die molekulardisperse Lycopinlösung bei Temperaturen von 50°C bis 240°C herstellt und unmittelbar anschließend im Schritt b) mit der wäßrigen Lösung des Schutzkolloids versetzt, wobei sich eine Mischungstemperatur von etwa 35°C bis 80°C einstellt.

10. Verfahren zur Herstellung von stabilen, pulverförmigen Lycopin-Formulierungen, definiert gemäß Anspruch 1, **dadurch gekennzeichnet, dass** man kristallines Lycopin mit einem all-trans Isomerengehalt von mindestens 50% in einem wäßrigen Medium in Anwesenheit eines Schutzkolloids mahlt und die so erhaltene Lycopinsuspension für die Herstellung eines Trockenpulvers trocknet.

11. Verfahren nach Anspruch 10, **dadurch gekennzeichnet, dass** der Kristallinitätsgrad von Lycopin in der pulverförmigen Formulierung durch Variation der Mahldauer und/oder Mahltemperatur eingestellt werden kann.

12. Verwendung der stabilen, pulverförmigen Lycopin-Formulierungen, definiert gemäß Anspruch 1 zur Herstellung von Nahrungsergänzungsmitteln sowie als Zusatz zu Lebensmitteln, Tierfuttermitteln, pharmazeutischen und kosmetischen Zubereitungen.

13. Verwendung der stabilen, pulverförmigen Lycopin-Formulierungen nach Anspruch 12 als Färbemittel.

## Claims

1. A stable, pulverulent lycopene formulation, comprising lycopene having a degree of crystallinity of greater than 20% and at least one protective colloid.

2. The lycopene formulation according to claim 1 comprising at least one protective colloid selected from the group of gelatin, fish gelatin, starch, dextrin, vegetable proteins, pectin, gum arabic, casein and/or caseinate.

3. The lycopene formulation according to claim 1 or 2, comprising at least fish gelatin as protective colloid.

4. The lycopene formulation according to any of claims 1 to 3, comprising lycopene having an all-trans isomer content of at least 50%.

5. The lycopene formulation according to any of claims 1 to 4, comprising 0.5 to 25% by weight of lycopene, 10 to 50% by weight of a protective colloid, 20 to 70% by weight of a plasticizer, 0 to 10% by weight of a stabilizer, all percentage data based on the dry mass of the powder.

6. The lycopene formulation according to any of claims 1 to 5, comprising lycopene having a particle size of less than 10 µm.

7. The lycopene formulation according to any of claims 1 to 6, which is water-dispersible.

8. A process for the preparation of stable, pulverulent lycopene formulations, as defined in claim 1, which comprises
a) preparing a molecularly disperse solution of lycopene, optionally together with an emulsifier and/or an edible oil in a water-miscible, organic solvent or a mixture of water and a water-miscible, organic solvent at temperatures of greater than 30°C,
b) mixing this solution with an aqueous solution of a protective colloid, the hydrophilic solvent component being transferred into the aqueous phase, and the hydrophobic phase of the lycopene resulting as a nanodisperse phase,
c) and freeing the formed dispersion from the solvent and the water for the preparation of a water-dispersible dry powder and, optionally, drying in the presence of a coating material.

9. The process according to claim 8, wherein, in step a), the molecularly disperse lycopene solution is prepared at temperatures from 50°C to 240°C and immediately subsequently mixed with the aqueous solution of the protective colloid in step b), a mixing temperature of approximately 35°C to 80°C being set.

10. A process for the preparation of stable, pulverulent lycopene formulations, as defined in claim 1, which comprises grinding crystalline lycopene having an all-trans isomer content of at least 50% in an aqueous medium in the presence of a protective colloid and drying the lycopene suspension thus obtained for the preparation of a dry powder.

11. The process according to claim 10, wherein the degree of crystallinity of lycopene in the pulverulent formulation can be adjusted by variation of the grinding time and/or grinding temperature.

12. The use of the stable, pulverulent lycopene formulations, as defined in claim 1, for the preparation of food supplements and as an additive to foodstuffs, animal feedstuffs, and pharmaceutical and cosmetic preparations.

13. The use of the stable, pulverulent lycopene formulations according to claim 12 as colorants.

## Revendications

1. Formulations stables, sous forme de poudre, de lycopène, contenant du lycopène présentant un degré de cristallinité supérieur à 20% et au moins un colloïde de protection.

2. Formulation de lycopène selon la revendication 1, contenant au moins un colloïde de protection choisi dans le groupe formé par la gélatine, la gélatine de poisson, l'amidon, la dextrine, les protéines végétales, la pectine, la gomme arabique, la caséine et/ou un caséinate.

3. Formulation de lycopène selon la revendication 1 ou 2, contenant au moins de la gélatine de poisson comme colloïde de protection.

4. Formulations de lycopène selon l'une quelconque des revendications 1 à 3, contenant du lycopène présentant une teneur en isomères all-trans d'au moins 50%.

5. Formulations de lycopène selon l'une quelconque des revendications 1 à 4, contenant 0,5 à 25% en poids de lycopène, 10 à 50% en poids d'un colloïde de protection, 20 à 70% en poids d'un plastifiant, 0 à 10% en poids d'un stabilisant, toutes les indications en pour cent se référant à la masse sèche de la poudre.

6. Formulations de lycopène selon l'une quelconque des revendications 1 à 5, contenant du lycopène présentant une grosseur de particule inférieure à 10 µm.

7. Formulations de lycopène selon l'une quelconque des revendications 1 à 6, **caractérisées en ce qu'**elles sont dispersibles dans l'eau.

8. Procédé pour la préparation de formulations stables, sous forme de poudre, de lycopène, définies selon la revendication 1, **caractérisé en ce que**
a) on prépare une solution dispersée moléculaire de lycopène, le cas échéant avec un émulsifiant et/ou une huile comestible, dans un solvant organique miscible à l'eau ou dans un mélange d'eau et d'un solvant organique miscible à l'eau, à des températures supérieures à 30°C,
b) on ajoute une solution aqueuse d'un colloïde de protection à cette solution, le composant hydrophile du solvant étant transféré dans la phase aqueuse et la phase hydrophobe du lycopène se formant sous forme de phase nanodispersée,
c) et, pour la préparation d'une poudre sèche dispersible dans l'eau, on libère la dispersion formée du solvant et de l'eau et on la sèche, le cas échéant en présence d'un matériau de revêtement.

9. Procédé selon la revendication 8, **caractérisé en ce que**, dans l'étape a), la solution dispersée moléculaire de lycopène est préparée à des températures de 50°C à 240°C et elle est additionnée, de manière immédiatement consécutive dans l'étape b), de la solution aqueuse du colloïde de protection, une température du mélange d'environ 35°C à 80°C se réglant.

10. Procédé pour la préparation de formulations stables, sous forme de poudre, de lycopène, définies selon la revendication 1, **caractérisé en ce qu'**on broie du lycopène cristallin présentant une teneur en isomères all-trans d'au moins 50% dans un milieu aqueux en présence d'un colloïde de protection et on sèche la suspension de lycopène ainsi obtenue pour la préparation d'une poudre sèche.

11. Procédé selon la revendication 10, **caractérisé en ce que** le degré de cristallinité du lycopène dans la formulation sous forme de poudre peut être réglé par variation de la durée de broyage et/ou de la température de broyage.

12. Utilisation des formulations stables, sous forme de poudre, de lycopène, définies selon la revendication 1 pour la préparation de compléments alimentaires ainsi que comme additif pour des aliments, des fourrages pour animaux, des préparations pharmaceutiques et cosmétiques.

13. Utilisation des formulations stables, sous forme de poudre, de lycopène selon la revendication 12 comme colorant.
